# EUROPÄISCHE PATENTSCHRIFT

(11) **EP 2 328 855 B1**
(45) Veröffentlichungstag und Bekanntmachung des Hinweises auf die Patenterteilung: **05.10.2016**
(21) Anmeldenummer: 09777511.8
(22) Anmeldetag: 29.07.2009
(51) Int. Cl.: C07C 29/152, C07C 31/04, B01J 8/06

(54) **VERFAHREN ZUR HERSTELLUNG VON METHANOL**
METHOD FOR THE PRODUCTION OF METHANOL
PROCÉDÉ DE FABRICATION DE MÉTHANOL

(30) Priorität: 30.09.2008 DE 102008049621
(43) Veröffentlichungstag der Anmeldung: 08.06.2011
(73) Patentinhaber: Air Liquide Global E&C Solutions Germany GmbH, 60439 Frankfurt am Main (DE)
(72) Erfinder: KOPETSCH, Hans, 61352 Bad Homburg (DE); HACKEL, Philipp, Marius, 61250 Usingen (DE); GRONEMANN, Veronika, 61184 Karben (DE); MORGENROTH, Rainer, 61381 Friedrichsdorf (DE)
(74) Vertreter: Keil & Schaafhausen Patent- und Rechtsanwälte PartGmbB
(86) Internationale Anmeldenummer: PCT/EP2009/005483
(87) Internationale Veröffentlichungsnummer: WO 2010/037440

(56) Entgegenhaltungen:
- EP-A1- 0 790 226
- US-B2- 7 144 923

## Beschreibung

Die Erfindung betrifft die Herstellung von Methanol aus einem Wasserstoff und Kohlenstoffoxide enthaltenden Synthesegas, wobei man das Synthesegas durch einen ersten, wassergekühlten Reaktor leitet, in welchem ein Teil der Kohlenstoffoxide katalytisch zu Methanol umgesetzt wird, wobei man das erhaltene, Synthesegas und Methanoldampf enthaltende Gemisch einem zweiten, gasgekühlten Reaktor zuführt, in welchem ein weiterer Teil der Kohlenstoffoxide katalytisch zu Methanol umgesetzt wird, wobei man Methanol von dem Synthesegas abtrennt und wobei man Synthesegas wieder zu dem ersten Reaktor zurückführt.

Ein derartiges Verfahren zur Herstellung von Methanol ist beispielsweise aus der EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem Kupferkatalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Bei instationären Anlagenzuständen und insbesondere beim unsachgemäßen Anfahren des Reaktors durch zu frühe Zugabe von frischem Synthesegas kann es bei dieser Verfahrensführung aber zu einer Kondensation des Methanol-Produktes im gasgekühlten zweiten Reaktor kommen, da sich die Reaktorwandtemperatur am Austritt dem Taupunkt des Produktgases annähern kann.

Aus der US 7,144,923 B2 ist ein Verfahren zur Methanolsynthese bekannt, bei welchem das Synthesegas zunächst eine adiabate Reaktorstufe durchläuft, in welcher ein Teil des Synthesegases zu Methanol umgesetzt wird, und anschließend zur weiteren Umsetzung einem gasgekühlten Reaktor zugeführt wird. Das in den gasgekühlten Reaktor eintretende Gas wird im Gleichstrom zum Frischgas geführt, um eine bessere Temperaturkontrolle des Synthesegases sowie eine bessere Wärmerückgewinnung zu erreichen. Bei Verwendung eines adiabaten ersten Reaktors wird jedoch der CO-Gehalt des verwendbaren Synthesegases beschränkt, da die Kontrolle der Exothermie im ersten Reaktor Probleme bereiten kann. Zudem ist es erforderlich, nach jeder Stufe des ersten Reaktors eine Kühlung des teilweise umgesetzten Synthesegases vorzunehmen, was den Aufbau des Reaktors kompliziert. Bei einem Vergleichsbeispiel sind die adiabatischen Reaktoren der ersten Reaktorstufe durch einen wassergekühlten Reaktor ersetzt, wobei die Kühlung des Synthesegases vor dem zweiten Reaktor beibehalten wird.

Aufgabe der Erfindung ist es, insbesondere bei Verwendung eines wassergekühlten ersten Reaktors die Kondensation von Methanol am Austritt des gasgekühlten zweiten Reaktors zuverlässig zu vermeiden.

Diese Aufgabe wird mit der Erfindung durch ein Verfahren mit den Merkmalen des Anspruchs 1 gelöst. Das Kühlgas durchströmt den zweiten Reaktor im Gleichstrom zu dem aus dem ersten Reaktor abgezogenen Gemisch. Dadurch erhöht sich der Abstand der Produkttemperatur vom Taupunkt am unteren Ende des Reaktors, so dass eine Methanolkondensation weitgehend ausgeschlossen wird. Durch Vermeidung einer Zweiphasenströmung kann zudem eine verbesserte Reaktorkontrolle erreicht werden. Gleichzeitig können Druckverluste bzw. Fluktuationen des Reaktordruckes zuverlässig vermieden werden.

Nachdem die Verwendung eines wassergekühlten ersten Reaktors den Einsatz eines "schärferen" Synthesegases mit höheren CO-Gehalten ermöglicht als bei einem adiabaten Reaktor gemäß der US 7,144,923 B2, können die Umsätze erhöht und eine höhere Konzentration von Methanol und Wasser als Koppelprodukt im Produktgas nach dem ersten Reaktor erreicht werden. Dies führt zu einem höheren Taupunkt, so dass die Kondensationsvermeidung im zweiten Reaktor noch wichtiger wird.

Gemäß einer bevorzugten Ausgestaltung des Verfahrens ist vorgesehen, dass als Kühlgas für den zweiten Reaktor Synthesegas verwendet wird, welches aus dem aus dem zweiten Reaktor abgezogenen Gemisch abgetrennt wurde. Damit lässt sich eine maximale Ausnutzung der durch den exothermen Reaktionsprozess erzielten Wärme erreichen.

Erfindungsgemäß wird das Kühlgas in den zweiten Reaktor mit einer Temperatur von 100 bis 120 °C eingeführt und aus diesem mit einer Temperatur von 205 bis 215 °C abgezogen.

In Weiterbildung der Erfindung wird das aus dem zweiten Reaktor abgezogene Kühlgas dann zu dem ersten Reaktor zurückgeführt, wobei das zurückgeführte Kühlgas dem ersten Reaktor zusammen mit frischem Synthesegas zuführt wird und der Anteil des frischen Synthesegases vorzugsweise etwa 15 bis 40 Vol.-% beträgt.

Die Erfindung erstreckt sich auch auf eine Verwendung einer Anlage zur Herstellung von Methanol aus einem Wasserstoff und Kohlenstoffoxide enthaltenden Synthesegas mit den Merkmalen des Anspruchs 6, die zur Durchführung des oben beschriebenen Verfahrens geeignet ist. Die Anlage umfasst einen ersten, wassergekühlten Reaktor, in welchem ein Teil der Kohlenstoffoxide katalytisch zu Methanol umgesetzt wird, einen zweiten, gasgekühlten Reaktor, welchem man das aus dem ersten Reaktor erhaltene Gasgemisch über eine Leitung zuführt und in welchem ein weiterer Teil der Kohlenstoffoxide zu Methanol umgesetzt wird, eine Abscheideeinrichtung zur Abtrennung des Methanols von dem Synthesegas sowie eine Rückführleitung zur Rückführung von Synthesegas zu dem ersten Reaktor. Erfindungsgemäß führt von der Abscheideeinrichtung eine Kühlleitung zu dem Eintritt des zweiten Reaktors, über welche dem zweiten Reaktor Synthesegas derart zugeführt wird, dass das Synthesegas im Gleichstrom zu dem aus dem ersten Reaktor erhaltenen Gasgemisch durch den zweiten Reaktor strömt.

Weiterbildungen, Vorteile und Anwendungsmöglichkeiten der Erfindung ergeben sich auch aus der nachfolgenden Beschreibung eines Ausführungsbeispiels und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen:
- Fig. 1: schematisch eine Anlage zur Durchführung des erfindungsgemäßen Verfahrens,
- Fig. 2: den Temperaturverlauf verschiedener Reaktorkomponenten über der normalisierten Länge des gasgekühlten Reaktors bei im Gegenstrom geführtem Kühlgas,
- Fig. 3: den Temperaturverlauf verschiedener Reaktorkomponenten über der normalisierten Länge des gasgekühlten Reaktors bei im Gleichstrom geführtem Kühlgas und
- Fig. 4: den Verlauf des Methanolgehaltes über der normalisierten Reaktorlänge für im Gleich- und Gegenstrom geführtes Kühlgas.

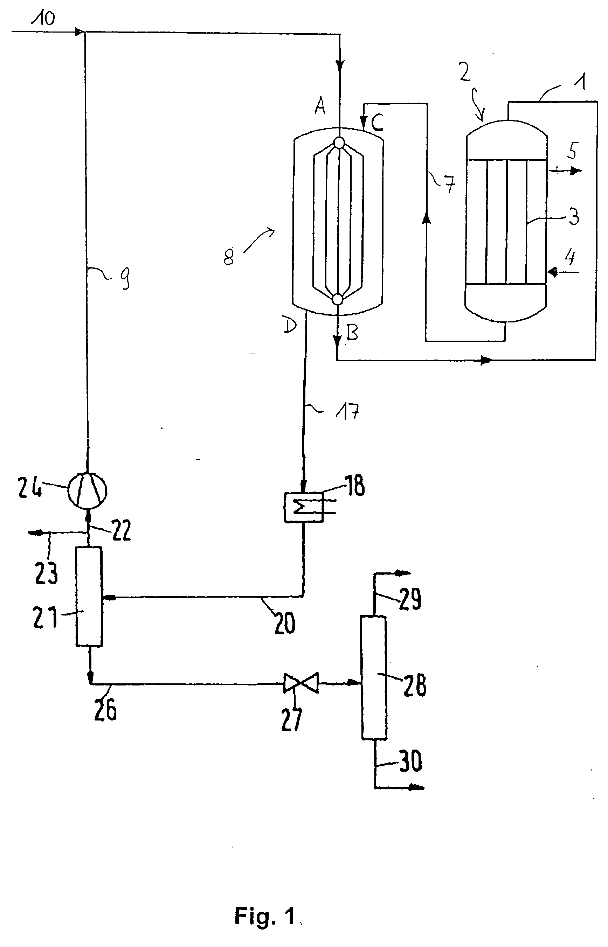

In der in Fig. 1 dargestellten Anlage wird ein Gemisch aus frischem und zurückgeführtem Synthesegas durch eine Leitung 1 in einen ersten Synthese-Reaktor 2 geführt. Dieser erste Reaktor 2 ist beispielsweise ein an sich bekannter Röhrenreaktor, in welchem beispielsweise ein Kupferkatalysator in Röhren 3 angeordnet ist. Als Kühlmittel dient unter erhöhtem Druck siedendes Wasser, das in der Leitung 4 herangeführt wird. Ein Gemisch aus siedendem Wasser und Wasserdampf zieht man in der Leitung 5 ab und führt es zur Energiegewinnung einer nicht dargestellten, an sich bekannten Dampftrommel zu.

Das in den ersten Reaktor 2 eintretende Synthesegas ist auf eine Temperatur > 220 °C vorgewärmt, da der Katalysator erst ab dieser Temperatur anspricht. Üblicherweise liegen die Gastemperatur am Eintritt des ersten Reaktors 2 bei 220 bis 280 °C und der Druck im Bereich von 2 bis 12 MPa (20 bis 120 bar), vorzugsweise im Bereich von 4 bis 10 MPa (40 bis 100 bar). Das Kühlmittel, das man über die Leitung 5 abzieht, hat üblicherweise eine Temperatur im Bereich von 240 bis 280 °C. In dem ersten Reaktor 2 werden in einer exothermen Reaktion je nach Zustand des Katalysators 40 bis 80 % der durch die Leitung 1 in den Reaktor 2 gegebenen Kohlenstoffoxide umgesetzt.

Aus dem ersten Reaktor 2 zieht man über die Leitung 7 ein erstes Gemisch, im Wesentlichen bestehend aus Synthesegas und Methanoldampf ab, wobei der Methanolgehalt 4 bis 10 Vol.-%, zumeist 5 bis 8 Vol.-%, beträgt. Dieses Gemisch leitet man in den zweiten Synthesereaktor 8, der beispielsweise ebenfalls als Röhrenreaktor mit einem Kupferkatalysator ausgestaltet ist. Der Katalysator kann wie bei dem ersten Reaktor 2 in den Röhren oder bevorzugt auf der Mantelseite vorgesehen sein.

Als Kühlmedium dient in dem zweiten Reaktor 8 Synthesegas, das über die Leitung 9 mit einer Temperatur von 80 bis 130 °C herangeführt wird und den zweiten Reaktor 8 im Gleichstrom mit dem aus dem ersten Reaktor 2 kommenden ersten Gemisch durchströmt.

Frisches Synthesegas, das man in einer an sich bekannten, hier nicht dargestellten Anlage erzeugt, wird in der Leitung 10 herangeführt und dem zurückzuführenden Synthesegas zugemischt. Die Temperatur des Kühlgases am Eintritt in den zweiten Reaktor 8 ergibt sich durch das Mischungsverhältnis zwischen rückgeführtem und frischem Synthesegas und wird umso niedriger gewählt je höher die Eintrittstemperatur des ersten Gemischs in den zweiten Reaktor 8 ist. Das als Kühlmittel dienende Synthesegas wird im zweiten Reaktor 8 vorgewärmt und strömt dann durch die Leitung 1 zum ersten Reaktor 2.

Das Synthesegas, das in den ersten Reaktor 2 eintritt, soll Wasserstoff und Kohlenstoffoxide etwa in folgenden Anteilen aufweisen:

| | |
|---|---|
| H₂ = | 40 bis 80 Vol.-% |
| CO = | 3 bis 15 Vol.-% und |
| CO₂ = | 1 bis 10 Vol.-%. |

Ein im Wesentlichen Synthesegas und Methanoldampf enthaltendes Produktgemisch (zweites Gemisch) verlässt den zweiten Reaktor 8 durch eine Leitung 17 und strömt durch einen indirekten Kühler 18, wobei Methanol kondensiert wird. Anschließend gibt man das Gemisch durch die Leitung 20 in einen ersten Abscheidebehälter 21, in welchem sich Gase und Flüssigkeit trennen. Die Gase werden durch die Leitung 22 abgezogen, wobei man einen Teil über eine Leitung 23 aus dem Verfahren entfernen kann. Mit Hilfe des Verdichters 24 führt man die Gase als zurückzuführendes Synthesegas (Recyclegas) über die Leitung 9 durch den zweiten Reaktor 8 und nach der hierdurch erfolgten Vorwärmung dann weiter in den ersten Reaktor 2.

Aus dem ersten Abscheidebehälter 21 zieht man über eine Leitung 26 Methanol enthaltende Flüssigkeit ab und führt die Flüssigkeit durch ein Entspannungsventil 27 zu einem zweiten Abscheidebehälter 28. Hieraus wird über eine Leitung 29 ein Restgas abgezogen, während man über die Leitung 30 Rohmethanol erhält, das nun in nicht dargestellter, an sich bekannter Weise destillativ gereinigt wird.

Es versteht sich, dass der Aufbau der Reaktoren 2, 8 als solchen nicht auf die oben beschriebenen Varianten beschränkt ist. Vielmehr sind auch Abwandlungen dieser Reaktoren möglich, beispielsweise so wie es in der EP 0 790 226 B1 beschrieben ist.

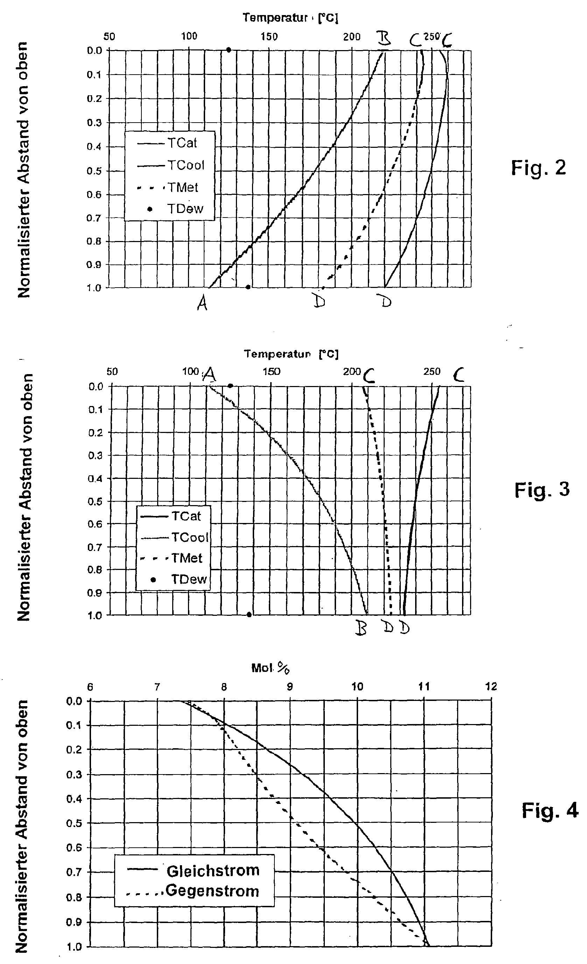

In den Diagrammen gemäß Fig. 2 und Fig. 3 sind beispielhaft die Temperaturverläufe des Katalysators (TCat), des Kühlgases (TCool) und der Kühlrohrwand (TMet) über der normalisierten Reaktorlänge für ein im Gegenstrom zu dem aus dem ersten Reaktor 2 kommenden ersten Gemisch geführtes Kühlgas (Fig. 2, vgl. EP 0 790 226 B1) bzw. für ein gemäß der vorliegenden Erfindung im Gleichstrom zu dem aus dem ersten Reaktor 2 kommenden ersten Gemisch geführtes Kühlgas (Fig. 3) dargestellt. Hierbei bezeichnen die Beginn- und Endpunkte der Temperaturkurven folgende Punkte im zweiten Reaktor:
A: Eintritt Kühlgas
B: Austritt Kühlgas
C: Eintritt erstes Gemisch (Reaktionsgas)
D: Austritt zweites Gemisch (Reaktionsgas)

TDew bezeichnet den Taupunkt des Produktgemisches am Eintritt und Austritt des zweiten Reaktors, der mit der Methanolkonzentration ansteigt.

Bei dem dargestellten Beispiel ergeben sich folgende Prozessdaten:

| | Gegenstrom (EP 0 790 226 B1) | Gleichstrom (Erfindung) |
|---|---|---|
| Kühlgas ein (A) [°C] | 112 | 112 |
| Kühlgas aus (B) [°C] | 218 | 209 |
| Reaktionsgas ein (C) [°C] | 255 | 255 |
| Reaktionsgas aus (D) [°C] | 220 | 233 |
| Abstand Taupunkt oben [°C] | 117 | 81 |
| Abstand Taupunkt unten [°C] | 45 | 86 |

Im normalen Betrieb beträgt der Abstand zwischen Taupunkt TDew des Reaktionsgases und der Temperatur der Kühlrohrwand TMet an der kältesten Stelle im unteren Teil des Reaktors einige 10 °C. Bei unsachgemäßer Inbetriebnahme der Reaktoren durch eine zu frühzeitige Zugabe von frischem Synthesegas in den Kreislauf kann bei der Gegenstromführung dieser sichere Abstand (45 °C) verloren gehen und eine Kondensation des Reaktionsgases auftreten.

Bei der Gleichstromführung verdoppelt sich dagegen nahezu der Abstand der Temperatur der Kühlrohrwand TMet von dem Taupunkt des Produktgemischs TDew im kritischen Bereich (Austritt des Produktgemischs) gegenüber der Gegenstromführung auf 86 °C. Damit ist auch beim Wiederanfahren des Synthesekreislaufs mit temperierten Reaktoren nach kurzzeitigem Anlagenausfall das Risiko einer Kondensation durch zu frühe Aufgabe von Synthesegas erheblich geringer.

Außerdem ergibt sich eine flachere Temperaturkurve über der Reaktorlänge mit geringeren Spitzentemperaturen. Dies ist vorteilhaft für die Laufzeitstabilität des Katalysators.

In Fig. 4 ist der Anstieg des Methanolgehaltes über der Reaktorlänge für im Gleich- und Gegenstrom geführtes Kühlgas dargestellt. Man erkennt, das beide Konzepte etwa die gleiche Gesamtproduktion liefern. Durch das flachere Temperaturprofil liefert allerdings die Gleichstromführung auf den ersten 2/3 der Reaktorlänge mehr Methanol als die Gegenstromführung.

### Bezugszeichenliste:

- 1: Leitung
- 2: erster Reaktor
- 3: Röhren
- 4: Leitung
- 5: Leitung
- 7: Leitung
- 8: zweiter Reaktor
- 9: Leitung
- 10: Leitung
- 17: Leitung
- 18: Kühler
- 20: Leitung
- 21: Abscheidebehälter
- 22: Leitung
- 23: Leitung
- 24: Verdichter
- 26: Leitung
- 27: Entspannungsventil
- 28: zweiter Abscheidebehälter
- 29: Leitung
- 30: Leitung

- A: Eintritt Kühlgas
- B: Austritt Kühlgas
- C: Eintritt erstes Gemisch (Reaktionsgas)
- D: Austritt zweites Gemisch (Reaktionsgas)

## Patentansprüche

1. Verfahren zur Herstellung von Methanol aus einem Wasserstoff und Kohlenstoffoxide enthaltenden Synthesegas, wobei man das Synthesegas durch einen ersten, wassergekühlten Reaktor leitet, in welchem ein Teil der Kohlenstoffoxide katalytisch zu Methanol umgesetzt wird, wobei man das erhaltene, Synthesegas und Methanoldampf enthaltende Gemisch einem zweiten, gasgekühlten Reaktor zuführt, in welchem ein weiterer Teil der Kohlenstoffoxide zu Methanol umgesetzt wird, wobei man Methanol von dem Synthesegas abtrennt und wobei man Synthesegas wieder zu dem ersten Reaktor zurückführt, **dadurch gekennzeichnet, dass** das Kühlgas den zweiten Reaktor im Gleichstrom zu dem aus dem ersten Reaktor abgezogenen Gemisch durchströmt und das Kühlgas in den zweiten Reaktor mit einer Temperatur von 100 bis 120 °C eingeführt wird.

2. Verfahren nach einem der vorhergehenden Ansprüche, **dadurch gekennzeichnet, dass** als Kühlgas für den zweiten Reaktor Synthesegas verwendet wird, welches aus dem aus dem zweiten Reaktor abgezogenen Gemisch abgetrennt wurde.

3. Verfahren nach einem der vorhergehenden Ansprüche, **dadurch gekennzeichnet, dass** das Kühlgas aus dem zweiten Reaktor mit einer Temperatur von 205 bis 215 °C abgezogen wird.

4. Verfahren nach einem der vorhergehenden Ansprüche, **dadurch gekennzeichnet, dass** das aus dem zweiten Reaktor abgezogene Kühlgas zu dem ersten Reaktor zurückgeführt wird.

5. Verfahren nach einem der vorhergehenden Ansprüche, **dadurch gekennzeichnet, dass** das zurückgeführte Kühlgas dem ersten Reaktor zusammen mit frischem Synthesegas zuführt wird, und dass der Anteil des frischen Synthesegases etwa 15 bis 40 Vol.-% beträgt.

6. Verwendung einer Anlage zur Herstellung von Methanol aus einem Wasserstoff und Kohlenstoffoxide enthaltenden Synthesegas zur Durchführung eines Verfahrens nach einem der vorhergehenden Ansprüche mit einem ersten, wassergekühlten Reaktor (2), in welchem ein Teil der Kohlenstoffoxide katalytisch zu Methanol umgesetzt wird, mit einem zweiten, gasgekühlten Reaktor (8), welchem man das aus dem ersten Reaktor (2) erhaltene Gasgemisch über eine Leitung (7) zuführt und in welchem ein weiterer Teil der Kohlenstoffoxide zu Methanol umgesetzt wird, mit einer Abscheideeinrichtung (21) zur Abtrennung des Methanols von dem Synthesegas und mit einer Rückführleitung (1) zur Rückführung von Synthesegas zu dem ersten Reaktor (2), **dadurch gekennzeichnet, dass** von der Abscheideeinrichtung (21) eine Kühlleitung (9) zu dem Eintritt des zweiten Reaktors (8) führt, über welche dem zweiten Reaktor (8) Synthesegas derart zugeführt wird, dass das Synthesegas im Gleichstrom zu dem aus dem ersten Reaktor (2) erhaltenen Gasgemisch durch den zweiten Reaktor (8) strömt.

## Claims

1. A process for producing methanol from a synthesis gas containing hydrogen and carbon oxides, wherein the synthesis gas is passed through a first, water-cooled reactor in which a part of the carbon oxides is catalytically converted to methanol, wherein the resulting mixture containing synthesis gas and methanol vapor is supplied to a second, gas-cooled reactor in which a further part of the carbon oxides is converted to methanol, wherein methanol is separated from the synthesis gas and wherein synthesis gas is recirculated to the first reactor, **characterized in that** the cooling gas flows through the second reactor co-current to the mixture withdrawn from the first reactor and that the cooling gas is introduced into the second reactor with a temperature of 100 to 120 °C.

2. The process according to any of the preceding claims, **characterized in that** as cooling gas for the second reactor synthesis gas is used, which has been separated from the mixture withdrawn from the second reactor.

3. The process according to any of the preceding claims, **characterized in that** the cooling gas is withdrawn from the second reactor with a temperature of 205 to 215 °C.

4. The process according to any of the preceding claims, **characterized in that** the cooling gas withdrawn from the second reactor is recirculated to the first reactor.

5. The process according to any of the preceding claims, **characterized in that** the recirculated cooling gas is supplied to the first reactor together with fresh synthesis gas and that the content of fresh synthesis gas is about 15 to 40 vol-%.

6. A plant for producing methanol from a synthesis gas containing hydrogen and carbon oxides for performing a process according to any of the preceding claims with a first, water-cooled reactor (2) in which a part of the carbon oxides is catalytically converted to methanol, with a second, gas-cooled reactor (8) to which the gas mixture obtained from the first reactor (2) is supplied via a conduit (7) and in which a further part of the carbon oxides is converted to methanol, with a separating means (21) for separating the methanol from the synthesis gas and with a return conduit (1) for recirculating synthesis gas to the first reactor (2), **characterized in that** from the separating means (21) a cooling conduit (9) leads to the inlet of the second reactor (8), via which synthesis gas is supplied to the second reactor (8) such that the synthesis gas flows through the second reactor (8) co-current to the gas mixture obtained from the first reactor (2).

## Revendications

1. Procédé de production de méthanol à partir d'un gaz de synthèse contenant de l'hydrogène et des oxydes de carbone, consistant à faire passer ledit gaz de synthèse par un premier réacteur refroidi à l'eau, dans lequel une partie des oxydes de carbone est transformée en méthanol en subissant un réaction catalytique, à obtenir ainsi un mélange contenant du gaz de synthèse et de la vapeur de méthanol puis à l'introduire dans un deuxième réacteur refroidi au gaz, dans lequel une partie supplémentaire des oxydes de carbone est transformée en méthanol en subissant une réaction, à séparer du méthanol du gaz de synthèse et à recycler du gaz de synthèse dans le premier réacteur, **caractérisé en ce que** le gaz de refroidissement traverse le deuxième réacteur à co-courant avec le mélange sortant du premier réacteur, et que le gaz de refroidissement est introduit dans le deuxième réacteur à une température comprise entre 100 et 120 °C.

2. Procédé selon l'une des revendications précédentes, **caractérisé en ce que** l'on utilise, en tant que gaz de refroidissement pour le deuxième réacteur, du gaz de synthèse qui vient d'être séparé du mélange sortant du deuxième réacteur.

3. Procédé selon l'une des revendications précédentes, **caractérisé en ce qu'**en sortant du deuxième réacteur, ledit gaz de refroidissement a une température comprise entre 205 et 215 °C.

4. Procédé selon l'une des revendications précédentes, **caractérisé en ce que** ledit gaz de refroidissement, qui vient de sortir du deuxième réacteur, est recyclé dans le premier réacteur.

5. Procédé selon l'une des revendications précédentes, **caractérisé en ce que** le gaz de refroidissement recyclé est introduit dans le premier réacteur ensemble avec du gaz de synthèse nouvellement mis en oeuvre et que la proportion de gaz de synthèse nouvellement mis en oeuvre est d'environ 15 à 40 % en volume.

6. Utilisation d'une installation de production de méthanol à partir d'un mélange contenant de l'hydrogène et du gaz de synthèse, laquelle permet de mettre en oeuvre un procédé selon l'une des revendications précédentes et laquelle est pourvue d'un premier réacteur (2) refroidi à l'eau, dans lequel une partie des oxydes de carbone est transformée en méthanol en subissant un réaction catalytique, d'un deuxième réacteur (8) dans lequel on introduit, à travers une conduite (7), le mélange gazeux obtenu à l'issue du premier réacteur (2) et dans lequel une partie supplémentaire des oxydes de carbone est transformée en méthanol en subissant une réaction, d'un dispositif de séparation (21) destiné à séparer le méthanol du gaz de synthèse, et d'une conduite de recyclage (1) destinée à recycler du gaz de synthèse dans le premier réacteur (2), **caractérisée en ce qu'**une conduite de refroidissement (9), laquelle relie le dispositif de séparation (21) à l'entrée du deuxième réacteur (8), permet d'introduire du gaz de synthèse dans le deuxième réacteur (8) de manière à ce que le gaz de synthèse traverse le deuxième réacteur (8) à co-courant avec le mélange gazeux obtenu à l'issue du premier réacteur (2).
